# FASCICULE DE BREVET EUROPEEN

(11) **EP 0 667 765 B1**
(45) Date de publication et mention de la délivrance du brevet: **10.03.1999**
(21) Numéro de dépôt: 94926269.5
(22) Date de dépôt: 02.09.1994
(51) Int. Cl.: A61K 6/083

(54) **PROTHESE DENTAIRE**
ZAHNPROTHESE
DENTAL PROSTHESIS

(30) Priorité: 03.09.1993 FR 9310537
(43) Date de publication de la demande: 23.08.1995
(73) Titulaire: DELAHAYE, Arnaud, F-92200 Neuilly-sur-Seine (FR)
(72) Inventeur: DELAHAYE, Arnaud, F-92200 Neuilly-sur-Seine (FR)
(74) Mandataire: Arnaud, Jean Pierre Alfred
(86) Numéro de dépôt international: FR9401036
(87) Numéro de publication internationale: WO9506453

(56) Documents cités:
- EP-A- 0 102 199
- DE-A- 2 403 211
- FR-A- 2 427 357
- US-A- 4 350 532
- US-A- 4 364 731
- DATABASE WPI Section Ch, Week 8709 Derwent Publications Ltd., London, GB; Class A96, AN 87-062515 & JP,A,62 019 160 (LION CORP (ASAG ) ASAHI GLASS KK) , 27 Janvier 1987

## Description

La présente invention concerne les prothèses dentaires, et des matériaux composites permettant leur réalisation.

Avant la description de l'invention, il convient de préciser la terminologie utilisée dans le présent mémoire et qui correspond à celle des hommes du métier.

Les soins des dents comprennent essentiellement le domaine des restaurations et reconstitutions proprement dentaires, et le domaine des reconstitutions prothétiques. Les restaurations désignent les obturations (de classes I à V), les inlays et les onlays, et les facettes.

Les reconstitutions prothétiques désignent les prothèses fixes ou amovibles. Ces reconstitutions ont une partie métallique et affectent le plus souvent plusieurs dents, bien qu'elles puissent aussi ne concerner qu'une seule dent. Elles sont fixées à une ou plusieurs dents. Elles peuvent être aussi fixées à un ou plusieurs implants. Ces reconstitutions sont aussi appelées "prothèses" dans le présent mémoire.

L'invention concerne les reconstitutions prothétiques de type fixe. Ces reconstitutions de type fixe comportent le plus souvent un support métallique, réalisé par coulée d'un métal ou d'un alliage, et une masse de reconstitution fixée au support et qui peut être formée de différents matériaux. Les matériaux les plus souvent utilisés pour ces reconstitutions fixes sont des matériaux céramiques. On utilise aussi des matières plastiques et parfois des matériaux composites.

Toutes ces reconstitutions fixes présentent des inconvénients.

Les reconstitutions céramiques ont une grande rigidité et une résistance à la flexion qui est insuffisante. Elles doivent être fixées sur des dents solidement consolidées. Cependant, le plus souvent, les personnes qui doivent porter une telle reconstitution de grande dimension ont et conservent des problèmes au niveau du parodonte (alvéolyse) avec ou non épaississement du ligament alvéolodentaire, et fréquemment une mobilité plus ou moins prononcée des dents. Le montage d'une telle reconstitution céramique sur des dents ainsi consolidées conduit à un taux d'échec important.

Les reconstitutions de matière plastique ont un grand intérêt au point de vue des reconstitutions des dents, car, lorsqu'une telle prothèse a été portée un certain temps, on s'aperçoit que la mobilité des dents est réduite, de même que l'alvéolyse et l'espace parodontal. Il est même parfois possible de réaliser une prothèse céramique fixée aux dents qui ont été ainsi consolidées par le port d'une prothèse de matière plastique pendant un certain temps. Cependant, une telle prothèse de matière plastique s'use très rapidement, et elle ne constitue donc pas une solution à long terme, mais seulement à titre provisoire ou temporaire.

Entre ces deux comportements extrêmes, on a déjà cherché à utiliser d'autres matériaux ayant un taux d'usure inférieur à celui de la matière plastique, tout en étant moins rigides et plus souples que la céramique.

Ainsi, le brevet français n° 2 427 357 relève les mauvaises propriétés de dureté, de résistance à la flexion et de résistance à l'abrasion des matières synthétiques utilisées à l'époque (1978). Ce brevet suggère l'utilisation d'une composition thermodurcissable destinée à l'art dentaire et qui peut être utilisée pour "certaines prothèses". La composition comporte "une charge renforçante" constituée de fibres de verre ou de microsphères de verre. La quantité de charge renforçante utilisée est comprise entre 1 et 50 %, et de préférence entre 15 et 40 %. Les propriétés mécaniques obtenues ne sont que peu supérieures à celles des matières synthétiques seules.

Ultérieurement, on a ainsi utilisé des matériaux composites, c'est-à-dire des matériaux ayant un liant polymère contenant une charge minérale, pour réaliser des reconstitutions. L'un des produits donnant actuellement les meilleurs résultats est le produit de marque "Dentacolor" (Heraeus-Kulzer). Cependant, ces produits ont une rigidité trop importante, et il arrive fréquemment qu'ils se fracturent, de la même manière que peuvent le faire les reconstitutions formées de céramique. En outre, leur résistance à l'usure est insuffisante pour les reconstitutions de très longue durée. On ne les a donc utilisés que très peu pour les reconstitutions.

La demande de brevet européen n° 102 199 concerne des compositions de restauration dentaire données comme ayant d'excellentes propriétés mécaniques. Cependant, ce document décrit une composition destinée uniquement aux restaurations dentaires, c'est-à-dire aux obturations et travaux analogues, et nullement une composition destinée aux prothèses. Rien ne suggère dans ce document la réalisation d'une prothèse.

Compte tenu des propriétés des reconstitutions fixes connues, dans des travaux préalables à l'invention, on a cherché quelles propriétés devait posséder un matériau permettant la réalisation de reconstitutions ou prothèses ne présentant ni les inconvénients des reconstitutions céramiques ni ceux des reconstitutions de matière plastique, et ayant leurs avantages. Plus précisément, les avantages des prothèses céramiques sont leur faible usure et leur résistance pendant une très longue durée, et ceux des prothèses de matière plastique sont leur souplesse et la consolidation du tissu de soutien des dents (parodonte) qu'elles engendrent. En outre, pour pouvoir être utilisé pour la réalisation de prothèse, un matériau doit posséder toutes les propriétés habituelles des matériaux utilisés en dentisterie, notamment au point de vue sanitaire, chimique et esthétique.

Après utilisation de matériaux céramiques et composites et de matières plastiques connus, on a ainsi déterminé selon l'invention que, pour permettre la réalisation de prothèses ayant à la fois les avantages des prothèses céramiques et ceux des prothèses de matière plastique, les propriétés que devait posséder un matériau étaient essentiellement des propriétés de résistance à la flexion et de dureté. On a alors déterminé selon l'invention qu'une masse de reconstitution formée à partir d'un matériau composite devait posséder une résistance à la flexion au moins égale à 100 MPa, et une dureté Vickers d'au moins 450 N/mm².

On s'est alors rendu compte que certains matériaux composites récemment proposés à la vente pour le seul domaine des restaurations (obturations, inlays, onlays, facettes) pouvaient aussi être utilisés, dans certaines conditions, pour la réalisation de prothèses ou reconstitutions prothétiques fixes, car ils permettent l'obtention des propriétés physiques originales nécessaires à la réalisation des prothèses. En outre, comme ces matériaux ont déjà été utilisés pour des restaurations, ils possèdent toutes les qualités sanitaires, chimiques et esthétiques nécessaires aux reconstitutions.

L'invention concerne des prothèses dentaires selon la revendication 1, et selon les sous-revendications 2 à 6 et des applications d'un matériau composite selon la revendication 7 et selon les sous-revendications 8 à 15.

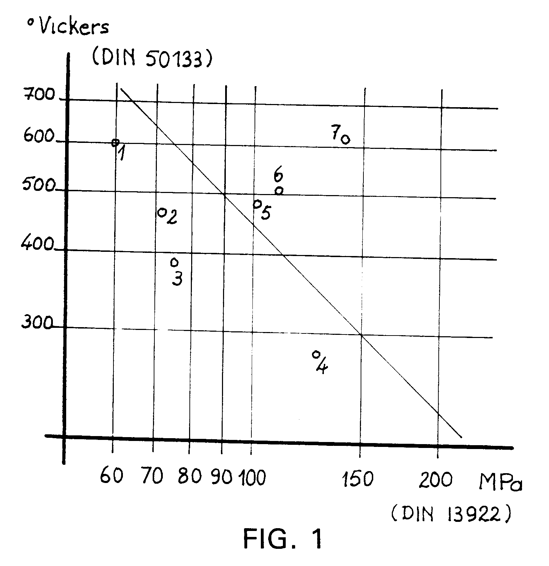

On considère maintenant plus en détail certaines caractéristiques essentielles de l'invention, en référence au dessin annexé dont la figure unique est un graphique indiquant le classement de différents matériaux en fonction de leurs propriétés de résistance à la flexion et de dureté.

On sait que les prothèses céramiques ont une dureté Vickers qui peut être supérieure à celle de la masse de reconstitution utilisée selon l'invention. Cependant, la résistance à la flexion de ces céramiques dentaires est très inférieure à celle de la masse de reconstitution utilisée selon l'invention. Au contraire, la matière plastique utilisée pour les prothèses a une résistance à la flexion bien supérieure à celle de la masse de reconstitution utilisée selon l'invention, mais sa dureté est extrêmement faible. On pourrait penser qu'il suffit de choisir un matériau dont les propriétés sont comprises entre celles de la céramique conventionnelle et celles de la matière plastique utilisée en dentisterie pour obtenir des propriétés convenant aux prothèses.

Le matériau composite connu "Dentacolor", cité précédemment, a été commercialisé à cet effet pour la formation de masses de reconstitution de prothèses. Il possède des propriétés comprises entre celles de la céramique conventionnelle et celles de la matière plastique utilisée en dentisterie. Cependant, ce matériau présente des inconvénients de la céramique (trop grande rigidité), sans les avantages de la matière plastique (consolidation du tissu de soutien des dents -parodonte). En fait, ce matériau composite "Dentacolor" a une résistance à la flexion bien inférieure à celle d'un matériau composite convenant à la mise en oeuvre de l'invention, et sa dureté est aussi bien inférieure à celle d'un tel matériau.

On considère maintenant des exemples de mise en oeuvre de différents matériaux céramiques et composites pour la réalisation de prothèses dentaires, dans les exemples qui suivent. Parmi ces exemples, seuls les exemples 5 à 7 correspondent à l'invention, les autres étant donnés à titre de comparaison.

### Exemple 1

On prépare des prothèses céramiques conventionnelle à l'aide de céramiques dentaires classiques "Duceram" et "Biodent" couramment utilisées, à base de feldspath et de silice, cuites à une température de l'ordre de 930 °C.

### Exemple 2

On prépare, par mise en oeuvre des procédures préconisées par le fabricant, des prothèses comportant une masse de reconstitution du matériau composite "Thermoresin LCII", contenant 70 % en poids d'une charge essentiellement à base de silice pyrogène. Son liant polymère est formé par polymérisation de diméthacrylate d'uréthanne et de diméthacrylate monomère. Ce produit est couramment disponible dans le commerce auprès de GC International Corp. Il existe en qualité opaque et en qualités transparente et de diverses couleurs.

### Exemple 3

On prépare, par mise en oeuvre des procédures préconisées par le fabricant, des prothèses comportant une masse de reconstitution du matériau composite "Dentacolor", contenant 51 % en poids d'une charge essentiellement constituée de silice pyrogène, de granulométrie moyenne de l'ordre de 0,04 µm. Son liant polymère est formé par polymérisation d'esters d'acide méthacrylique polyfonctionnel. Ce produit est couramment disponible dans le commerce auprès de Heraeus-Kulzer. Il existe en qualité opaque et en qualités transparente et de diverses couleurs.

### Exemple 4

On prépare, par mise en oeuvre des procédures préconisées par le fabricant, des prothèses comportant une masse de reconstitution du matériau composite "Coltène Brilliant". Ce produit est couramment disponible dans le commerce auprès de Coltène Whaledent. Il existe en qualité opaque et en qualités transparente et de diverses couleurs.

### Exemple 5

On prépare, par mise en oeuvre des procédures préconisées par le fabricant, des prothèses comportant une masse de reconstitution du matériau composite "Cesead", contenant 82 % en poids d'une charge de particules minérales très fines et de composés organiques. Son liant polymère est photopolymérisable à froid. Ce produit est fabriqué par Kuraray Co. Ltd. et est couramment disponible dans le commerce. Il existe en qualité opaque et en qualités transparente et de diverses couleurs.

### Exemple 6

On prépare, par mise en oeuvre des procédures préconisées par le fabricant, des prothèses comportant une masse de reconstitution du matériau composite "Charisma". Il contient 77 % en poids de charge minérale contenant dix parties d'un verre de borosilicate de baryum et d'aluminium pour une partie de silice traitée par un silane. La dimension particulaire du verre de borosilicate est comprise entre 0,02 et 2 µm. Ce produit est couramment disponible dans le commerce auprès de Heraeus-Kulzer. Il existe en qualité opaque et en qualités transparente et de diverses couleurs.

### Exemple 7

On prépare des prothèses analogues à celles de l'exemple 6, et on leur fait subir une recuisson par mise en oeuvre des procédures préconisées par le fabricant.

### Exemple 8

On prépare, par mise en oeuvre des procédures habituelles aux prothésistes, des prothèses comportant une masse de reconstitution d'une matière plastique essentiellement formée de polymère de méthacrylate couramment utilisé pour la fabrication de prothèses de matière plastique.

### Détermination des propriétés mécaniques

On a alors déterminé les propriétés mécaniques de chacune des masses de reconstitution des prothèses sur des éprouvettes préparées chacune en même temps et dans les mêmes conditions que la masse de reconstitution correspondante. Ces propriétés mécaniques étaient la résistance à la flexion, déterminée selon la norme DIN 13922, et la dureté Vickers déterminée selon la norme DIN 50133. On a aussi calculé un paramètre égal au produit de cette résistance à la flexion et de cette dureté Vickers.

On a obtenu les résultats du tableau suivant.

| Propriétés mécaniques des exemples | | | | | |
|---|---|---|---|---|---|
| | (1) | (2) | (3) | | (4) |
| Matériau | MPa | N/mm² | %pds | %vol | k(MPa)² |
| Céramique de l'exemple 1 | 50-70 | 500-700 | - | - | ≈36 |
| Composite de l'exemple 2 | 71,5 | 464 | 70 | 55 | 33,2 |
| Composite de l'exemple 3 | 75 | 385 | 51 | 35 | 28,9 |
| Composite de l'exemple 4 | 127 | 278 | ? | ? | 35,3 |
| Composite de l'exemple 5 | 101 | 480 | 82 | 64 | 48,5 |
| Composite de l'exemple 6 | 110 | 510 | 77 | 60 | 56,1 |
| Composite (recuit) de l'exemple 7 | 140 | 620 | 77 | 60 | 86,8 |
| Composite de l'exemple 8 | >1000 | <30 | - | - | <30 |

| | | | | | |
|---|---|---|---|---|---|
| (1) Résistance à la flexion (DIN 13922) | | | | | |
| (2) Dureté Vickers (DIN 50133) | | | | | |
| (3) Teneur en charges minérales, en poids en en volume | | | | | |
| (4) Produit des valeurs des colonnes (1) et (2) | | | | | |

La figure unique représente, sur un graphique logarithmique-logarithmique, les résultats des exemples 1 à 7. La droite inclinée représente la valeur 45 k(MPa)² du produit de la colonne (4).

Les valeurs des différentes propriétés indiquées dans le tableau montrent que, si l'on classe les différents matériaux dans l'ordre croissant de résistance à la flexion, les matériaux composites des exemples 5 et 6 ont une dureté particulièrement élevée, même à l'état non recuit. Si l'on classe au contraire ces matériaux dans l'ordre des duretés, on note que les matériaux composites des exemples 5 et 6 ont une résistance à la flexion exceptionnellement élevée, même à l'état non recuit.

### Détermination des résultats cliniques

Des essais cliniques ont été effectués, pour la validation de l'invention, sur les prothèses réalisées dans les exemples précédents.

Dans le cas des prothèses céramiques de l'exemple 1, on a observé des problèmes au niveau du parodonte (alvéolyse), avec ou non épaississement du ligament alvéolo-dentaire. On a parfois souvent une mobilité plus ou moins prononcée des dents.

Dans le cas des prothèses de matériaux composites des exemples 2 et 3, on a observé des problèmes de fracture des prothéses.

En outre, dans le cas des prothèses de matériaux composites des exemples 3 et surtout 4, on a rapidement constaté une usure significative.

Dans le cas des prothèses de matériaux composites des exemples 5 à 7, on a observé dans la majorité des cas une consolidation, c'est-à-dire une diminution de l'espace entre le dent et l'alvéole, c'est-à-dire un retour à la normale de la physiologie du ligament alvéolo-dentaire, et donc une suppression de l'alvéolyse et une diminution de l'espace parodontal, comme dans le cas de l'utilisation des prothèses de matière plastique. L'usure des masses de reconstitution est pratiquement négligeable. On n'avait encore jamais observé ces propriétés avec des prothèses de céramique conventionnelle, ni avec des prothèses des matériaux composites connus jusqu'à présent.

### Conclusions des essais mécaniques et cliniques

Les résultats des essais mécaniques et cliniques montrent qu'une dureté élevée seule est inopérante (problèmes posés par les prothèses céramiques), qu'une grande résistance à la flexion seule ne convient pas non plus (problèmes posés par les prothèses de matière plastique), et qu'un compromis entre la dureté et la résistance à le flexion (problèmes posés par les prothèses des exemples 2, 3 et 4) ne suffit pas non plus. En fait, comme l'indique la colonne (4) du tableau, les prothèses donnant les résultats avantageux de l'invention présente un produit de la résistance à la flexion et de la dureté qui est nettement supérieur à celui de tous les matériaux qui ont été utilisés auparavant pour la réalisation des masses de reconstitution des prothèses dentaires. L'exemple selon l'invention possédant le plus faible produit de ces propriétés donne une valeur supérieure de la moitié environ au moins à celles de tous les matériaux antérieurement utilisés pour ces prothèses (céramiques, matériaux composites tels que "Dentacolor" et matières plastiques). La figure unique montre clairement comment les matériaux selon l'invention (au-dessus de la droite inclinée) se distinguent des matériaux utilisés auparavant pour les masses de reconstitution des prothèses dentaires (au-dessous de la droite inclinée).

En conséquence, selon l'invention, c'est la combinaison des propriétés de résistance à la flexion (au moins égale à 100 MPa) et de dureté (au moins égale à 450 N/mm²) du matériau composite qui est la caractéristique essentielle.

Les essais cliniques réalisés sur des restaurations dentaires par les fabricants des matériaux composites des exemples 5 à 7 ont montré que ces matériaux avaient d'excellentes propriétés de résistance à l'usure et à l'abrasion et conduisaient à une occlusion non traumatisante. Ces matériaux possèdent ainsi les meilleures propriétés qu'on attend d'un matériau composite utilisé pour les restaurations de dents : très grande résistance à l'usure, même après de longues périodes (deux ans), usure la plus faible parmi les matériaux composites présents sur le marché, excellentes caractéristiques de surface et notamment excellentes propriétés de polissage, grande commodité de mise en oeuvre, excellent aspect esthétique, à la fois par la coloration et la profondeur des teintes, etc. Toutes ces propriétés sont également valables dans le cas des reconstitutions. Elles sont au moins équivalentes à celles qui sont obtenues avec les céramiques conventionnelles dans le cas des reconstitutions.

Les prothèses selon l'invention donnent donc une combinaison originale et particulièrement avantageuse de propriétés, puisqu'elles possèdent les avantages des prothèses céramiques (longue durée, aspect esthétique) et ceux des prothèses de matière plastique (reconstitution du ligament alvéolo-dentaire, suppression de l'alvéolyse et fermeture de l'espace parodontal).

On décrit maintenant plus en détail, à titre illustratif, la réalisation d'une prothèse de l'exemple 6 selon l'invention, constituant un bridge complet.

D'abord, tous les éléments métalliques du bridge sont montés à la cire, de manière classique (cire coulée). Ensuite, l'armature métallique est transformée en métal par la méthode de la cire perdue. L'armature est nettoyée au jet de vapeur, puis par sablage, toujours de manière classique. La surface est ensuite préparée pour la formation d'une couche d'accrochage à base de silice, par exemple par le procédé "Silicoater MD" commercialisé par Heraeus-Kulzer. Après la formation de la couche d'accrochage, le procédé comprend la formation d'une première couche, puis généralement d'une seconde, constituées d'un opacifiant destiné à cacher le métal. L'opacifiant de ces couches peut être un produit opacifiant opaque "Dentacolor", l'épaisseur de cette couche étant habituellement de quelques dixièmes de millimètre. Ensuite, le matériau composite "Charisma" est appliqué par couches successives pour la formation de la masse de la reconstitution. A cet effet, le prothésiste utilise différentes teintes (pour la couleur et la transparence) de "Charisma" appliquées successivement en couches d'épaisseur toujours inférieure à 2 mm. Chaque couche est polymérisée par irradiation lumineuse. La dernière couche est destinée à former l'émail. Dans un mode de réalisation avantageux, la prothèse subi une recuisson par irradiation prolongée. Après polymérisation, la prothèse est soumise à une finition classique, essentiellement par polissage.

La prothèse selon l'invention est donc réalisée par un procédé essentiellement classique que connaissent déjà les prothésistes. Cependant, les propriétés obtenues sont bien supérieures à celles des prothèses formées des matériaux composites classiques et, de façon générale, de tous les matériaux habituellement utilisés pour les prothèses dentaires. De même, la prothèse peut être mise en place par les procédés classiques, par exemple par scellement sur des dents ou vissage sur des onlays de reconstitution dentaire ou par scellement ou vissage sur des implants. Elle donne alors au patient un confort qui ne peut être obtenu avec aucun des matériaux connus.

L'invention concerne donc l'application d'une nouvelle catégorie de matériaux composites, permettant la formation de masses de reconstitution ayant une résistance à la flexion au moins égale à 100 MPa, et une dureté Vickers au moins égale à 450 N/mm², utilisés aujourd'hui pour les restaurations dentaires, à la réalisation de reconstitutions. Dans cette nouvelle application, ces matériaux composites donnent des propriétés qui constituent une combinaison originale qui n'est obtenue avec aucun des matériaux connus pour la réalisation des reconstitutions dentaires. Cette combinaison originale de propriétés n'est aucunement suggérée dans l'application normale de ces matériaux composites aux restaurations habituelles. Il faut noter que ces propriétés avantageuses sont encore améliorées parce que le métal du support, après sa coulée, ne subit plus de traitement à température élevée qui pourrait réduire ses propriétés, comme dans le cas des prothèses céramiques dont la cuisson est réalisée à des températures de l'ordre de 930 °C. En particulier, ce métal conserve ses propriétés avantageuses d'élasticité.

Bien qu'on ait décrit l'invention en référence à l'utilisation de certains matériaux composites, elle s'applique à l'utilisation d'autres matériaux du moment qu'ils possèdent la combinaison de propriétés de résistance à la flexion et de dureté selon l'invention. Bien que l'invention ne soit pas limitée par une quelconque explication théorique des résultats obtenus, il semble, comme l'indique la colonne (3) du tableau, qu'il est important que le matériau composite contienne une très grande quantité de charge minérale.

Il est bien entendu que l'invention n'a été décrite est représentée qu'à titre d'exemple préférentiel.

## Revendications

1. Prothèse dentaire, du type qui comprend un support métallique, et au moins une masse de reconstitution fixée au support métallique, la masse de reconstitution étant formée, pour sa plus grande partie au moins, d'un matériau composite contenant un liant polymère dans lequel est dispersée une charge minérale, caractérisée en ce que la masse de reconstitution a une résistance à la flexion au moins égale à 100 MPa, et elle possède une dureté Vickers qui est au moins égale à 450 N/mm².

2. Prothèse selon la revendication 1, caractérisée en ce qu'elle comprend une couche d'accrochage assurant la fixation de la masse au support métallique.

3. Prothèse selon l'une des revendications 1 et 2, caractérisée en ce que le liant polymère de la masse de reconstitution est avantageusement formé par polymérisation de monomères contenant des méthacrylates esters.

4. Prothèse selon l'une quelconque des revendications 1 à 3, caractérisée en ce que la charge minérale contenue dans le liant polymère contient essentiellement un verre de borosilicate finement broyé.

5. Prothèse selon la revendication 4, caractérisée en ce que la dimension particulaire moyenne du verre de borosilicate est comprise entre 0,02 et 2 µm.

6. Prothèse selon l'une quelconque des revendications précédentes, caractérisée en ce que la quantité de charge minérale soit au moins égale à 55 % en volume de la masse de reconstitution.

7. Application d'un matériau composite à la réalisation d'une prothèse dentaire qui comprend une masse de reconstitution fixée au support, du type qui comprend la formation de la plus grande partie au moins de la masse de reconstitution à l'aide d'un matériau composite contenant un liant polymère dans lequel est dispersée une charge minérale, caractérisée en ce qu'elle comprend la sélection, pour la formation de la plus grande partie au moins de la masse de reconstitution, d'un matériau composite tel que, après polymérisation du matériau composite, la masse de reconstitution possède à la fois une résistance à la flexion au moins égale à 100 MPa, et une dureté Vickers au moins égale à 450 N/mm².

8. Application selon la revendication 7, caractérisée en ce qu'elle comprend la formation d'une couche d'accrochage sur le support avant la formation de la masse de reconstitution.

9. Application selon l'une des revendications 7 et 8, caractérisée en ce que la sélection d'un matériau composite comprend la sélection d'un matériau composite dont le polymère destiné à former le liant de la masse de reconstitution est formé de monomères contenant des méthacrylates esters.

10. Application selon l'une quelconque des revendications 7 à 9, caractérisée en ce que la sélection d'un matériau composite comprend la sélection d'un matériau composite dont la charge contient essentiellement un verre de borosilicate finement broyé.

11. Application selon la revendication 10, caractérisée en ce que la sélection d'un matériau composite comprend la sélection d'un matériau composite dont la charge de verre de borosilicate a la dimension particulaire moyenne comprise entre 0,02 et 2 µm.

12. Application selon l'une quelconque des revendications 7 à 11, caractérisée en ce que la sélection d'un matériau composite comprend la sélection d'un matériau composite ayant une quantité de charge minérale au moins égale à 55 % en volume de la masse de reconstitution formée par le matériau.

13. Application selon la revendication 9, caractérisée en ce que la formation de la masse de reconstitution comprend la photopolymérisation de monomères contenant des méthacrylates esters, puis une recuisson.

14. Application selon la revendication 13, caractérisée en ce que la sélection d'un matériau composite comprend la sélection d'un matériau composite tel que la masse de reconstitution obtenue a une résistance à la flexion de l'ordre de 110 MPa au moins, lorsqu'elle n'a pas été recuite, et de l'ordre de 140 MPa au moins lorsqu'elle a été recuite.

15. Application selon l'une des revendications 13 et 14, caractérisée en ce que la sélection d'un matériau composite comprend la sélection d'un matériau composite tel que la dureté de la masse de reconstitution est de l'ordre de 500 N/mm² au moins lorsqu'elle n'a pas été recuite et de l'ordre de 600 N/mm² au moins lorsqu'elle a été recuite.

## Claims

1. Dental prosthesis, of the type comprising a metal support, and at least one reconstruction mass fixed to the metal support, at least the major portion of the reconstruction mass being formed of a composite material containing a polymer binder having an inorganic filler dispersed therein, being characterized in that the reconstruction mass has a bending strength of not less than 100 MPa, and possesses a Vickers hardness of not less than 450 N/mm².

2. Prosthesis according to claim 1, characterized in that it includes a coupling layer for fixing the mass to the metal support.

3. Prosthesis according to any of claims 1 and 2, characterized in that the polymer binder of the reconstruction mass is advantageously constituted by polymerizing monomers containing methacrylate esters.

4. Prosthesis according to anyone of claims 1 to 3, characterized in that the inorganic filler contained in the polymer binder essentially comprises a finely ground borosilicate glass.

5. Prosthesis according to claim 4, characterized in that the average particle size of the borosilicate glass lies in the range 0.02 µm to 2 µm.

6. Prosthesis according to any preceding claim, characterized in that the quantity of inorganic filler is not less than 55 % by volume of the reconstruction mass.

7. Use of a composite material for providing a dental prosthesis which includes a reconstruction mass fixed to a support, of the type including forming at least the major portion of the reconstruction mass with said composite material containing a polymer binder having an inorganic filler dispersed therein, characterized by selecting, for forming at least the major portion of the reconstruction mass, a composite material such that, after polymerizing of said composite material, the reconstruction mass has both a bending strength of not less than 100 MPa and a Vickers hardness of not less than 450 N/mm².

8. Use according to claim 7, characterized in that it includes forming a coupling layer on the support before forming the reconstruction mass.

9. Use according to any of claims 7 and 8, characterized in that selecting a composite material includes selecting a composite material whose the polymer forming the binder of the reconstruction mass is constituted by monomers containing methacrylate esters.

10. Use according to any of claims 7 to 9, characterized in that selecting a composite material includes selecting a composite material whose the inorganic filler essentially comprises a finely ground borosilicate glass.

11. Use according to claim 10, characterized in that selecting a composite material includes selecting a composite material whose the borosilicate glass has an average particle size lying in the range 0.02 µm to 2 µm.

12. Use according to any of claims 7 to 11, characterized in that selecting a composite material includes selecting a composite material whose the quantity of inorganic filler is not less than 55 % by volume of the reconstruction mass.

13. Use according to claim 9, characterized in that forming the reconstruction mass includes photopolymerizing monomers containing methacrylate esters, and then tempering.

14. Use according to claim 13, characterized in that selecting a composite material includes selecting a composite material such that the obtained reconstruction mass has a bending strength not less than at least about 110 MPa, prior to tempering, and not less than at least about 140 MPa after it has been tempered.

15. Use according to any of claims 13 and 14, characterized in that selecting a composite material includes selecting a composite material such that the hardness of the reconstruction mass is not less than at least about 500 N/mm² prior to tempering, and of at least about 600 N/mm² after it has been tempered.

## Patentansprüche

1. Zahnprothese, enthaltend ein metallisches Tragelement sowie wenigstens eine an diesem metallischen Tragelement befestigte Aufbaumasse, wobei die Aufbaumasse wenigstens zum größten Teil aus einem Verbundmaterial besteht, das ein Polymerbindemittel enthält, in welchem ein mineralisches Füllmittel dispergiert ist, wobei die Zahnprothese dadurch gekennzeichnet ist, daß die Aufbaumasse eine Biegefestigkeit von wenigstens 100 MPa und eine Härte nach Vickers von wenigstens 450 N/mm² aufweist.

2. Prothese nach Anspruch 1, dadurch gekennzeichnet, daß sie eine Halterungsschicht zur Befestigung der Masse am metallischen Tragelement enthält.

3. Prothese nach einem der Ansprüche 1 und 2, dadurch gekennzeichnet, daß das Polymerbindemittel der Aufbaumasse vorzugsweise durch Polymerisation aus Methakrylsäureestern enthaltenden Monomeren gebildet wird.

4. Prothese nach einem der Ansprüche 1 bis 3, dadurch gekennzeichnet, daß das im Polymerbindemittel enthaltene mineralische Füllmittel im wesentlichen ein fein vermahlenes Borsilikatglas umfaßt.

5. Prothese nach Anspruch 4, dadurch gekennzeichnet, daß die durchschnittliche Partikelgröße des Borsilikatglases zwischen 0,02 und 2 µm beträgt.

6. Prothese nach einem der vorangegangenen Ansprüche, dadurch gekennzeichnet, daß die Menge des mineralischen Füllmittels wenigstens 55 Vol.-% der Aufbaumasse beträgt.

7. Einsatz eines Verbundmaterials bei der Herstellung einer Zahnprothese, welche eine an einem Tragelement befestigte Aufbaumasse enthält, wobei der Einsatz des Verbundmaterials die Herstellung wenigstens des größten Teils der Aufbaumasse unter Verwendung eines Verbundmaterials umfaßt, das ein Polymerbindemittel enthält, in welchem ein mineralisches Füllmaterial dispergiert ist, wobei der Einsatz des Verbundmaterials dadurch gekennzeichnet ist, daß dabei ein Verbundmaterial für die Herstellung wenigstens des größten Teils der Aufbaumasse so ausgewählt wird, daß nach der Polymerisation des Verbundmaterials die Aufbaumasse gleichzeitig eine Biegefestigkeit von wenigstens 100 MPa und eine Härte nach Vickers von wenigstens 450 N/mm² aufweist.

8. Einsatz eines Verbundmaterials nach Anspruch 7, dadurch gekennzeichnet, daß der Einsatz des Verbundmaterials vor der Herstellung der Aufbaumasse die Bildung einer Halterungsschicht auf dem Tragelement umfaßt.

9. Einsatz eines Verbundmaterials nach Anspruch 7, dadurch gekennzeichnet, daß die Auswahl eines Verbundmaterials die Auswahl eines Verbundmaterials umfaßt, bei dem das zur Bildung des Bindemittels für die Aufbaumasse dienende Polymer aus Monomeren hergestellt wird, welche Methakrylsäureester enthalten.

10. Einsatz eines Verbundmaterials nach einem der Ansprüche 7 bis 9, dadurch gekennzeichnet, daß die Auswahl eines Verbundmaterials die Auswahl eines Verbundmaterials umfaßt, dessen Füllmaterial im wesentlichen ein fein vermahlenes Borsilikatglas enthält.

11. Einsatz eines Verbundmaterials nach Anspruch 10, dadurch gekennzeichnet, daß die Auswahl eines Verbundmaterials die Auswahl eines Verbundmaterials umfaßt, bei dem das aus Borsilikatglas bestehende Füllmittel eine durchschnittliche Partikelgröße von zwischen 0,02 und 2 µm aufweist.

12. Einsatz eines Verbundmaterials nach einem der Ansprüche 7 bis 11, dadurch gekennzeichnet, daß die Auswahl eines Verbundmaterials die Auswahl eines Verbundmaterials umfaßt, bei dem die Menge an Füllmittel wenigstens 55 Vol.-% der aus dem Material gebildeten Aufbaumasse beträgt.

13. Einsatz eines Verbundmaterials nach Anspruch 9, dadurch gekennzeichnet, daß die Herstellung der Aufbaumasse die Photopolymerisation von Monomeren, welche Methakrylsäureester enthalten, und ein darauffolgendes Nachhärten umfaßt.

14. Einsatz eines Verbundmaterials nach Anspruch 13, dadurch gekennzeichnet, daß die Auswahl eines Verbundmaterials die Auswahl eines Verbundmaterials auf eine Weise umfaßt, daß die hergestellte Aufbaumasse ohne Nachhärten einen Biegewiderstand im Bereich von wenigstens 110 MPa und bei erfolgtem Nachhärten einen Biegewiderstand im Bereich von wenigstens 140 MPa aufweist.

15. Einsatz eines Verbundmaterials nach einem der Ansprüche 13 und 14, dadurch gekennzeichnet, daß die Auswahl eines Verbundmaterials die Auswahl eines Verbundmaterials auf eine Weise umfaßt, daß die Härte der Aufbaumasse ohne Nachhärten wenigstens im Bereich von 500 N/mm² und bei erfolgtem Nachhärten im Bereich von wenigstens 600 N/mm² liegt.
